(19) Europäisches Patentamt
European Patent Office
Office européen des brevets

(11) Veröffentlichungsnummer: **0 563 666 A1**

(12) **EUROPÄISCHE PATENTANMELDUNG**

(21) Anmeldenummer: **93104152.9**

(22) Anmeldetag: **15.03.93**

(51) Int. Cl.[5]: **C07D 209/08**, C07D 209/10, C07D 209/12, C07D 209/24, B41M 5/136, B41M 5/20, B41M 5/30

(30) Priorität: **27.03.92 DE 4209919**

(43) Veröffentlichungstag der Anmeldung: **06.10.93 Patentblatt 93/40**

(84) Benannte Vertragsstaaten: **CH DE FR GB LI**

(71) Anmelder: **BAYER AG**
**D-51368 Leverkusen(DE)**

(72) Erfinder: **Berneth, Horst, Dr.**
**Erfurter Strasse 1**
**W-5090 Leverkusen 1(DE)**
Erfinder: **Köcher, Matthias, Dr.**
**Eichen 46**
**W-5063 Overath(DE)**

(54) **Toner für die Elektrofotografie, neue Indolderivate und Verfahren zu deren Herstellung.**

(57) Toner für die Elektrofotografie, die als Ladungskontrollsubstanzen Indolderivate der Formel (I) enthalten

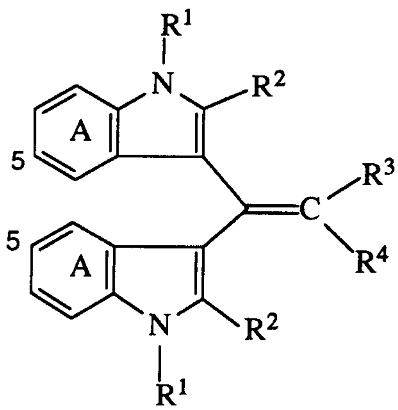

(I),

in der

$R^1$, $R^2$ und $R^3$ unabhängig voneinander für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl oder gegebenenfalls substituiertes $C_6$-$C_{10}$-Aryl stehen,

die Ringe A gegebenenfalls mit einem Benzolring anelliert und/oder gegebenenfalls mit bis zu 2 Resten aus der Gruppe $C_1$-$C_8$-Alkyl, $C_1$-$C_8$-Alkoxy, Halogen, Cyano, Nitro oder $C_6$-$C_{10}$-Aryl substituiert sind und

$R^4$ für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl, gegebenenfalls substituiertes $C_6$-$C_{10}$-Aryl oder einen Rest der Formeln (II) oder (III) steht

EP 0 563 666 A1

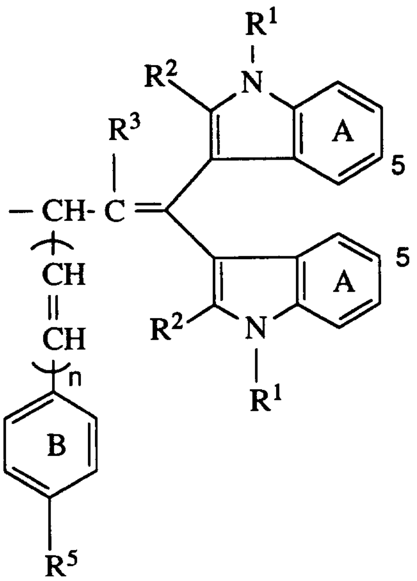

(II)

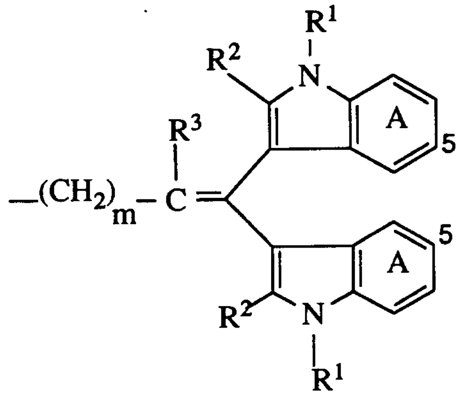

(III),

in denen

| | |
|---|---|
| $R^1$, $R^2$, $R^3$ und A | die unmittelbar zuvor angegebene Bedeutung haben, |
| $R^5$ | für Wasserstoff, gegebenenfalls verzweigtes $C_1$-$C_8$-Alkyl, $C_5$-$C_7$-Cycloalkyl, gegebenenfalls verzweigtes $C_1$-$C_8$-Alkoxy, Halogen, Nitro, Cyano, $C_1$-$C_8$-Alkoxycarbonyl, $C_6$-$C_{10}$-Aryl oder Di-$C_1$-$C_8$-Alkylamino und |
| m | für eine ganze Zahl von 1 bis 14 und |
| n | für Null oder 1 stehen und |

der Ring B unabhängig von den Ringen A außer mit $R^5$ gegebenenfalls wie die Ringe A anelliert und/oder substituiert ist. Diese Indolderivate sind teilweise neu.

Die vorliegende Erfindung betrifft Toner für die Elektrofotografie, die teilweise neue Indolderivate enthalten, und die Herstellung der neuen Indolderivate.

Toner werden bei der Entwicklung elektrostatischer Ladungsbilder in der Elektrofotografie, Elektrografie und Ionografie und magnetostatischer Ladungsbilder in der Magnetografie verwendet. Von diesen Verfahren hat die Elektrofotografie (siehe z.B. US-PS 2 297 691, US-PS 3 666 363 und US-PS 4 071 361) die größte Bedeutung erlangt.

Bei dieser Methode wird auf einem Fotoleiter durch Belichtung des Originals oder Umsetzung einer digitalisierten Bildinformation mit Hilfe eines Lasers, eines LED-Arrays oder einer LCS-Einheit ein virtuelles Bild in Form von elektrischen Ladungen erzeugt. Die Entwicklung dieses Ladungsbildes erfolgt durch Kontakt mit einem Toner. Bei dem Toner handelt es sich im allgemeinen um ein Pulver pigmentierter Teilchen, die eine definierte Partikelgröße und definierte elektrische Eigenschaften haben.

Der das Bild aufbauende Toner wird von der Oberfläche des Fotoleiters auf das Trägermaterial (z.B. Papier oder Folie) übertragen und dort fixiert.

Die Entwicklung des Ladungsbildes auf der Oberfläche des Fotoleiters kann nach dem Einkomponentenverfahren oder nach dem Zweikomponentenverfahren erfolgen.

Beim Zweikomponentenverfahren (siehe z.B. US-PS 2 874 063) werden Tonerteilchen mit einer Größe von 5 bis 30 µm und 80 bis 90 Gew.-% Tonerharz, 5 bis 15 Gew.-% Pigment und geringe Mengen Additive enthaltend, durch mechanische Agitation mit magnetischen Trägerteilchen (z.B. aus Roheisen, beschichtetem Roheisen oder Ferriten) mit einer Größe von 30 bis 200 µm triboelektrisch aufgeladen und bilden so eine Schicht auf der Oberfläche der entgegengesetzt aufgeladenen Trägerteilchen. Die Trägerteilchen werden mit Hilfe eines Magnetfeldes an einer rotierenden Walze (sog. magnetische Bürste) radial ausgerichtet und in permanenten Kontakt mit dem ebenfalls rotierenden Fotoleiter gebracht. Die an den Trägerteilchen haftenden Tonerpartikel werden auf die dem virtuellen Ladungsbild entsprechenden entgegengesetzt aufgeladenen Stellen übertragen.

Beim Einkomponentenverfahren (siehe z.B. US-PS 3 909 258) werden Carrier-Toner-Hybridteilchen eingesetzt. Diese haben eine Größe von 5 bis 30 µm und enthalten 20 bis 65 Gew.-% magnetische Pigmente, 30 bis 80 Gew.-% Tonerharz und geringe Mengen Additive. Der Toner wird elektrisch oder triboelektrisch vorgeladen und durch ein radial orientiertes Magnetfeld an der Oberfläche einer rotierenden Walze in räumliche Nähe des Fotoleiters gebracht. Die Tonerteilchen werden auf die dem virtuellen Ladungsbild entsprechenden geladenen Stellen übertragen.

Bei der Fixierung des auf das Trägermaterial (z.B. Papier oder Folie) übertragenen Toners haben sich in der Praxis die Flashfixierung und die Heißwalzenfixierung durchgesetzt.

Bei der Flashfixierung wird der Toner mittels einer Blitzlichtlampe (Xenonlampe) schnell und kontaktlos auf dem Trägermaterial aufgeheizt, so daß er in die Trägeroberfläche einschmilzt.

Bei der Heißwalzenfixierung wird der Träger mit dem anhaftenden Toner in Kontakt mit einer beheizten Walze gebracht, die den Toner unter Druck und Hitze mit der Trägeroberfläche dauerhaft verbindet.

Die Bildqualität der Reproduktion hängt immer wesentlich von den Eigenschaften des Toners ab, wobei die elektrischen Eigenschaften, insbesondere die triboelektrische Aufladung und die Leitfähigkeit für sämtliche Transferprozesse bedeutsam sind. Eine Beeinflussung der elektrischen Eigenschaften ist durch spezielle Additive (z.B. sogenannte Ladungskontrollsubstanzen) oder durch Variation der Pigmente (z.B. Ruß) möglich.

Für eine gute Bildqualität muß das Ladungsniveau und die Leitfähigkeit des Toners der Polarität und Vorspannung des Fotoleiters, der Transferspannung zum Übertrag auf das Trägermaterial und der jeweils verwendeten Vorrichtung (z.B. Kopierer oder Laserprinter) exakt angepaßt sein. Außerdem muß sichergestellt sein, daß die Ladungen und die Leitfähigkeiten einzelner Tonerpartikel nicht oder nicht wesentlich voneinander abweichen.

Wenn die Ladung zu niedrig oder die Leitfähigkeit zu hoch ist, erfolgt keine ausreichende Übertragung von der magnetischen Bürste auf den Fotoleiter, die Entwicklung wird unspezifisch (was zu Unschärfen führt) und der Transfer auf das Trägermaterial wird unzureichend (Anhäufung von sog. "waste-toner").

Bei zu hoher Ladung sind aufgrund ausgeprägter Haftung des Tonerpulvers an der magnetischen Bürste und am Fotoleiter die Transfervorgänge stark behindert und in ihrer Effektivität nicht ausreichend.

Wenn das Tonerpulver Teilchen mit unterschiedlichem Ladungsniveau enthält werden höher geladene Teilchen bevorzugt übertragen und im noch nicht übertragenen Toner erfolgt eine Anreicherung von Teilchen mit niederer Ladung. Hieraus resultiert eine unzulängliche Performance bei langer Betriebsdauer. Äußerst störend wirken Partikel mit umgekehrter Polarität (sogenannte "wrong-sign-particles").

Für den störungslosen Ablauf elektrofotografischer Prozesse ist es auch erforderlich, daß das Ladungsniveau und die Leitfähigkeit der Tonerpartikel möglichst unabhängig von klimatischen Einflüssen ist, z.B. von der Temperatur und der Luftfeuchtigkeit.

Man versucht Tonern die gewünschten Eigenschaften zu verleihen, indem man ihnen sogenannte Ladungskontrollsubstanzen zusetzt, beispielsweise 1 bis 3 Gew.-% spezielle Ammonium- oder Pyridiniumsalze` Metallkomplexe und/oder spezielle Pigmente (siehe US-PS 38 93 935, 39 44 493, 40 07 293, 40 79 014, 42 98 672, 43 38 390, 43 94 439, 44 93 883 und die DE-OS 36 04 827).

Man kann dabei so verfahren, daß man beispielsweise 5 bis 15 Gew.-% übliche Pigmente, 0,5 bis 3 Gew.-% Ladungskontrollsubstanzen und gegebenenfalls weitere Additive (z.B. 1 bis 3 Gew.-% Polyolefinwachse zur Steigerung der adhäsiven Eigenschaften) in das Harz einknetet, dann fein mahlt, nachfolgend zur Einstellung einer definierten Teilchengröße mehrere Sichtungsschritte vornimmt und abschließend mit hochdisperser Kieselsäure oder ähnlichen Produkten die gewünschten Pulverfließeigenschaften einstellt.

Für die Qualität des so erhaltenen Toners ist die Einheitlichkeit der einzelnen Tonerpartikel von größter Wichtigkeit. Daher werden hohe Anforderung an die Dispergierbarkeit der Additive, insbesondere der Ladungskontrollsubstanzen gestellt.

Des weiteren dürfen die verwendeten Ladungskontrollsubstanzen keine chemischen Wechselwirkungen mit Geräteteilen (z.B. Fotoleiter oder Fixierwalze) eingehen, die zu irreversiblen Verschmutzungen und Beschädigungen der betroffenen Teile führen würden. Hierbei ist insbesondere im Hinblick auf die beheizten Fixierwalzen eine hohe Thermostabilität der Ladungskontrollsubstanzen gefordert.

Zusätzlich zu diesen vielfältigen Anforderungen an Ladungskontrollsubstanzen müssen diese bei der Herstellung von farbigen Fotokopien und farbigen Bild reproduktionen eine gute Transparenz und möglichst nur eine geringe oder am besten gar keine Eigenfarbe aufweisen.

Die bisher bekannten farblosen, positiv aufladenden Ladungskontrollreagenzien besitzen ionische Strukturelemente, die naturgemäß hohe Affinitäten gegenüber Wasser aufweisen. Hieraus resultiert eine Abhängigkeit der Aufladungshöhe von den klimatischen Bedingungen, insbesondere der Luftfeuchtigkeit.

Die Toner der vorliegenden Erfindung enthalten farblose, positiv aufladende Ladungskontrollreagenzien, die nicht ionisch aufgebaut und trotzdem gut wirksam sind. Die Wasseraufnahme solcher Toner ist auf ein Minimum reduziert und die Klimaabhängigkeit der Toner hervorragend niedrig.

Es wurden nun Toner für die Elektrofotografie gefunden, die dadurch gekennzeichnet sind, daß sie als Ladungskontrollsubstanzen Indolderivate der Formel (I) enthalten

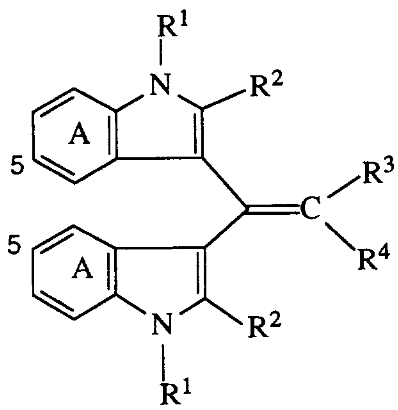

(I),

in der

$R^1$, $R^2$ und $R^3$ unabhängig voneinander für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl oder gegebenenfalls substituiertes $C_6$-$C_{10}$-Aryl stehen,

die Ringe A gegebenenfalls mit einem Benzolring anelliert und/oder gegebenenfalls mit bis zu 2 Resten aus der Gruppe $C_1$-$C_8$-Alkyl, $C_1$-$C_8$-Alkoxy, Halogen, Cyano, Nitro oder $C_6$-$C_{10}$-Aryl substituiert sind und

$R^4$ für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl, gegebenenfalls substituiertes $C_6$-$C_{10}$-Aryl oder einen Rest der Formeln (II) oder (III) steht

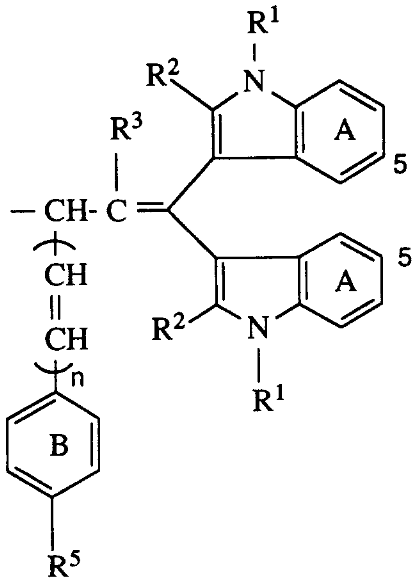

(II)

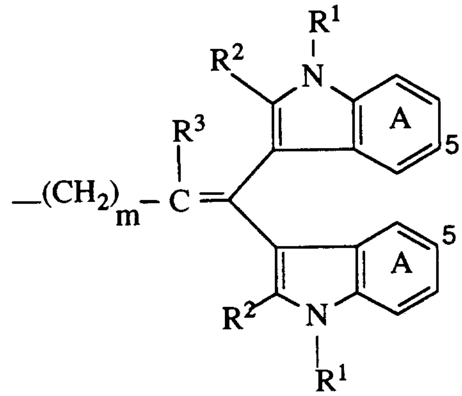

(III),

in denen

$R^1$, $R^2$, $R^3$ und A — die unmittelbar zuvor angegebene Bedeutung haben,

$R^5$ — für Wasserstoff, gegebenenfalls verzweigtes $C_1$-$C_8$-Alkyl, $C_5$-$C_7$-Cycloalkyl, gegebenenfalls verzweigtes $C_1$-$C_8$-Alkoxy, Halogen, Nitro, Cyano, $C_1$-$C_8$-Alkoxycarbonyl, $C_6$-$C_{10}$-Aryl oder Di-$C_1$-$C_8$-Alkylamino und

m — für eine ganze Zahl von 1 bis 14 und

n — für Null oder 1 stehen und

der Ring B unabhängig von den Ringen A außer mit $R^5$ gegebenenfalls wie die Ringe A anelliert und/oder substituiert ist.

Bevorzugte Toner für die Elektrofotografie sind dadurch gekennzeichnet, daß in Formel (I)

$R^1$ — für Wasserstoff, gegebenenfalls verzweigtes und/oder durch bis 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl oder Phenyl steht,

$R^2$ — für gegebenenfalls verzweigtes und/oder durch bis zu 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl oder gegebenenfalls durch bis zu 2 Substituenten aus der Grupe $C_1$-$C_4$-Alkyl, $C_1$-$C_4$-Alkoxy, Chlor, Nitro und Cyano substituiertes Phenyl steht,

$R^3$ — für Wasserstoff, Methyl oder Ethyl steht,

die Ringe A gegebenenfalls mit einem Benzolring anelliert oder gegebenenfalls mit bis zu 2 Resten aus der Gruppe Methyl, Ethyl, Methoxy, Chlor, Nitro, Cyano oder Phenyl substituiert sind und

$R^4$ — für Wasserstoff, gegebenenfalls verzweigtes und/oder durch bis zu 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl, gegebenenfalls durch bis zu 2 Substituenten aus der Gruppe $C_1$-$C_4$-Alkyl, $C_1$-$C_4$-Alkoxy, Chlor, Brom, Nitro und Cyano substituiertes Phenyl oder einen Rest der Formeln (II) oder (III) steht, in denen

$R^1$, $R^2$, $R^3$ und A — die unmittelbar zuvor angegebene Bedeutung haben,

$R^5$ — für Wasserstoff, gegebenenfalls verzweigtes $C_1$-$C_4$-Alkyl, Cycloalkyl, gegebenenfalls verzweigtes $C_1$-$C_4$-Alkoxy, Chlor, Brom, Nitro, Cyano, $C_1$-$C_4$-Alkoxycarbonyl, Phenyl oder Di-$C_1$-$C_4$-alkylamino und

m — für eine ganze Zahl von 1 bis 14 und

n — für Null oder 1 stehen und

der Ring B außer mit $R^5$ gegebenenfalls mit einem Benzolring anelliert oder gegebenenfalls bis zu zwei Resten aus der Gruppe Methyl, Ethyl, Methoxy, Chlor, Nitro, Cyano oder Phenyl substituiert ist.

Besonders bevorzugte Toner für die Elektrofotografie sind dadurch gekennzeichnet, daß in der Formel (I)

$R^1$ — für gegebenenfalls verzweigtes, unsubstituiertes $C_1$-$C_{22}$-Alkyl oder Benzyl steht,

$R^2$ für gegebenenfalls verzweigtes, unsubstituiertes $C_1$-$C_8$-Alkyl oder gegebenenfalls durch bis zu 2 Substituenten aus der Gruppe $C_1$-$C_4$-Alkyl, $C_1$-$C_4$-Alkoxy oder Chlor substituiertes Phenyl steht,

$R^3$ für Wasserstoff steht,

die Ringe A nicht anelliert, nicht substituiert oder in 5-Stellung durch Methyl, Methoxy oder Chlor substituiert sind und

$R^4$ für Wasserstoff, gegebenenfalls verzweigtes unsubstituiertes $C_1$-$C_{22}$-Alkyl oder einen Rest der Formeln (II) oder (III) steht,

in denen

$R^1$, $R^2$, $R^3$ und A die unmittelbar zuvor angegebene Bedeutung haben,

$R^5$ für Wasserstoff, gegebenenfalls verzweigtes $C_1$-$C_4$-Alkyl, gegebenenfalls verzweigtes $C_1$-$C_4$-Alkoxy, Chlor, Nitro, Cyano, Methoxycarbonyl oder Di-$C_1$-$C_4$-alkylamino und

m für eine ganze Zahl von 2 bis 8 und

n für Null oder 1 stehen und

der Ring B nicht anelliert oder außer mit $R^5$ nicht substituiert ist.

Bei den Indolderivaten der Formel (I) sind mehrfach mit gleichen Symbolen bezeichnete Substituenten in der Regel untereinander gleich, also beispielsweise die beiden Substituenten $R^1$ in den beiden in Formel (I) vorhandenen Indolstrukturen, sowie auch in den beiden Indolstrukturen, die zusätzlich vorhanden sind, wenn $R^4$ für einen Rest der Formeln (II) oder (III) steht. Es ist jedoch auch denkbar, daß mehrfach mit dem gleichen Symbol gekennzeichnete Substituenten innerhalb des jeweils angegebenen Bedeutungsumfangs verschieden voneinander sind.

Erfindungsgemäße Toner können einen oder mehrere Indolderivate der Formel (I) enthalten, beispielsweise insgesamt 0,5 bis 3 Gew.-% dieser Indolderivate und im übrigen wie üblich zusammengesetzt sein. Beispielsweise können erfindungsgemäße Toner 40 bis 90 Gew.-% Tonerharz, 4 bis 10 Gew.-% Pigmente (z.B. Ruß), 0 bis 5 Gew.-% Polyolefine und gegebenenfalls 30 bis 60 Gew.-% magnetische Pigmente enthalten, wobei die Summe aller dieser Bestandteile 100 Gew.-% ergibt.

Erfindungsgemäße Toner zeigen eine hohe Aufladung, eine gute Langzeitstabilität im Gerätetest und eine hohe Unempfindlichkeit gegen Klimatische Einflüsse, insbesondere gegen Luftfeuchtigkeit.

Indolderivate der Formel (1) sind zum Teil bekannte Verbindungen (s. DE-OS 3 738 237, US-PS 5 001 105 und US-PS 4 870 050).

Es wurden auch neue Indolderivate gefunden, die als Ladungskontrollsubstanzen für Toner geeignet sind. Diese sind auch Gegenstand der vorliegenden Erfindung und entsprechen den Formeln (IV), (V) und (VI)

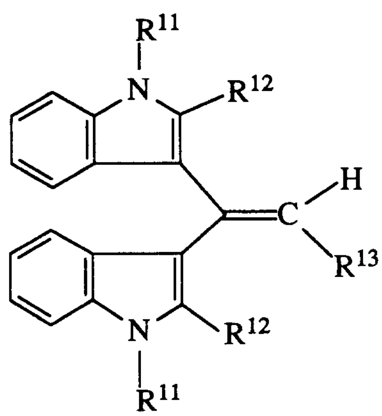

(IV)

in der

$R^{11}$ für gegebenenfalls verzweigtes und/oder gegebenenfalls durch bis zu 3 Substituenten aus der Gruppe Chlor, $C_1$-$C_4$-Alkoxy und Cyano substituiertes $C_5$-$C_{22}$-Alkyl steht und

entweder

$R^{12}$ für Phenyl, Tolyl, Anisyl oder Chlorphenyl und

$R^{13}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{16}$-Alkyl stehen

oder

$R^{12}$ für Tolyl, Anisyl oder Chlorphenyl und

$R^{13}$ für Wasserstoff oder gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{16}$-Alkyl stehen,

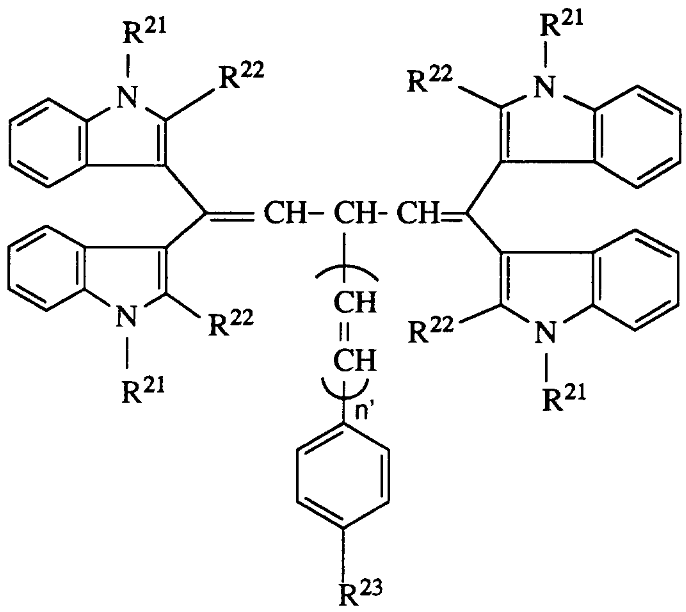
(V),

in der

$R^{21}$ für gegebenenfalls verzweigtes und/oder gegebenenfalls durch bis zu 3 Substituenten aus der Gruppe Chlor, $C_1$-$C_4$-Alkoxy und Cyano substituiertes $C_5$-$C_{22}$-Alkyl,

$R^{22}$ für Phenyl, Tolyl, Anisyl oder Chlorphenyl,

$R^{23}$ für Wasserstoff, Methyl, Methoxy, Chlor, Nitro oder Dimethylamino und

n' für Null oder 1 stehen und

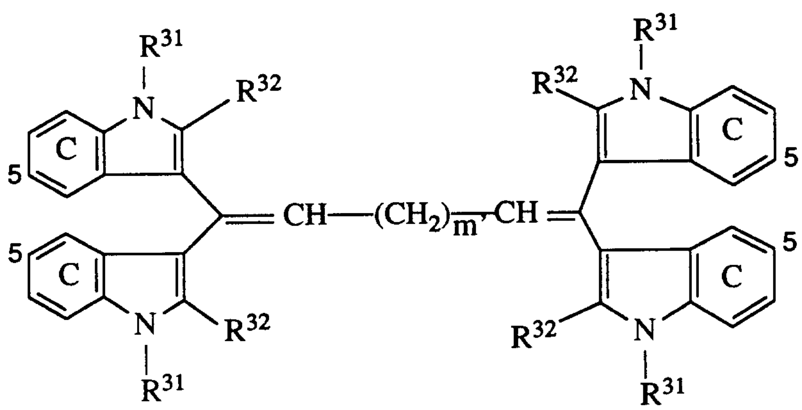
(VI),

in der

$R^{31}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{22}$-Alkyl oder für Benzyl, Phenethyl, Cyclohexyl oder Cyclopentyl,

$R^{32}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_8$-Alkyl oder für gegebenenfalls durch bis zu 3 Substituenten aus der Gruppe Chlor, $C_1$-$C_4$-Alkyl, $C_1$-$C_4$-Alkoxy und Cyano substituiertes Phenyl und

m' für eine ganze Zahl von 1 bis 14 stehen,

wobei die Ringe C gegebenenfalls mit einem Benzolring annelliert und/oder gegebenenfalls mit bis zu 2 Resten aus der Gruppe $C_1$-$C_8$-Aryl substituiert sind.

In Formel (IV) steht $R^{11}$ bevorzugt für gegebenenfalls verzweigtes, unsubstituiertes $C_5$-$C_{22}$-Alkyl, insbesondere $C_5$-$C_{12}$-Alkyl. Im Falle $R^{12}$ = Tolyl, Anisyl oder Chlorphenyl steht $R^{13}$ vorzugsweise für Wasserstoff oder unsubstituiertes $C_1$-$C_4$-Alkyl. Im Falle $R^{12}$ = Phenyl steht $R^{13}$ vorzugsweise für unsubstituiertes $C_1$-$C_4$-Alkyl.

In Formel (V) steht $R^{21}$ vorzugsweise für gegebenenfalls verzweigtes, unsubstituiertes $C_5$-$C_{22}$-Alkyl, insbesondere $C_5$-$C_{12}$-Alkyl.

In Formel (VI) steht $R^{31}$ vorzugsweise für gegebenenfalls verzweigtes, unsubstituiertes $C_1$-$C_{22}$-Alkyl, insbesondere $C_1$-$C_{12}$-Alkyl, $R^{32}$ vorzugsweise für gegebenenfalls verzweigtes unsubstituiertes $C_1$-$C_8$-Alkyl, Phenyl, Chlorphenyl, Tolyl oder Phenethyl und m' vorzugsweise für eine ganze Zahl von 2 bis 8. Weiterhin sind in Formel (VI) die Ringe C vorzugsweise nicht annelliert, nicht substituiert oder in 5-Stellung durch $C_1$-$C_4$-Alkyl, $C_1$-$C_4$-Alkoxy oder Chlor substituiert.

Bei den Indolderivaten der Formeln (IV), (V) und (VI) sind mehrfach mit den gleichen Symbolen bezeichnete Substituenten in der Regel untereinander gleich, also beispielsweise beiden Substituenten $R^{11}$ in Formel (IV) oder die vier Substituenten $R^{22}$ in Formel (V). Es ist jedoch auch denkbar, daß mehrfach mit dem gleichen Symbol bezeichnete Substituenten innerhalb des jeweils angegebenen Bedeutungsumfangs verschieden voneinander sind.

Die neuen Indolderivate der Formeln (IV), (V) und (VI) können hergestellt werden, indem man Verbindungen der Formeln (IV), (V) bzw. (VI), bei denen $R^{11}$, $R^{21}$ bzw. $R^{31}$ für Wasserstoff steht, mit einem Alkylierungsmittel der Formel (VII) umsetzt

$R^{41}$-X (VII),

in der

$R^{41}$ die bei der Formel (IV) für $R^{11}$, die bei Formel (V) für $R^{21}$ oder die bei Formel (VI) für $R^{31}$ angegebene Bedeutung hat und

X für Chlor, Brom, Iod, $OSO_2OR^{41}$, $OSO_2CH_3$, $OSO_2C_6H_5$, $OSO_2C_6H_4$-p-$CH_3$ oder $OSO_2C_6H_4$-p-Cl steht.

Indolderivate der Formeln (IV) und (V) mit $R^{11}$ bzw. $R^{21}$ = Wasserstoff und deren Herstellung sind beispielsweise aus der DE-OS 3 738 237 bekannt oder können in analoger Weise dazu erhalten werden. Analog dazu lassen sich auch Indolderivate der Formel (VI) mit $R^{31}$ = Wasserstoff erhalten.

Die Umsetzung der Indolderivate der Formeln (IV), (V) und (VI) mit $R^{11}$, $R^{21}$ bzw. $R^{31}$ = Wasserstoff mit Alkylierungsmittel der Formel (VII) kann beispielsweise in einem Zweiphasensystem durchgeführt werden. Die eine Phase stellt dann eine organische Phase dar, die andere eine wäßrige Alkalilösung. Als organische Phase kommen mit Wasser nicht mischbare Lösungsmittel in Frage. Beispiele sind substituierte und unsubstituierte Kohlenwasserstoffe, wie Benzol, Toluol, Xylol, Anisol, Chlorbenzol, Dichlorbenzol, $C_6$-$C_{20}$-Aliphaten und Dichlormethan. Die wäßrige Alkalilösung kann beispielsweise eine Lösung von Lithium-, Natrium- oder Kaliumhydroxid oder Natrium- oder Kaliumcarbonat in Wasser sein. Vorteilhaft wird dem Zweiphasensystem ein sogenannter Phasentransferkatalysator zugesetzt, bei dem es sich um organische Ammoniumsalze oder Kronenether handeln kann, beispielsweise um Trimethylbenzylammoniumchlorid, Tetrabutylammoniumbromid, Trimethyloctylammoniumbromid, Trimethylcaprylammoniumbromid oder 18-Krone-6. Der Zusatz katalytischer Mengen eines Iodids kann vorteilhaft sein, beispielsweise der Zusatz von Natrium- oder Kaliumiodid. Es ist vorteilhaft die Umsetzung unter intensivem Rühren durchzuführen, beispielsweise bei Temperaturen zwischen Raumtemperatur und der Siedetemperatur des Mediums. Bevorzugte Reaktionstemperaturen liegen im Bereich von 40 bis 100 °C. Nach beendeter Umsetzung können die beiden Phasen getrennt und die hergestellten Indolderivate aus der organischen Phase, beispielsweise durch Entfernung des Lösungsmittels, isoliert werden.

Das Alkylierungsmittel der Formel (VII) setzt man üblicherweise etwa im stöchiometrischen Verhältnis ein, d.h. a) bei der Alkylierung von zwei N-Atomen (Herstellung von Verbindungen der Formel (IV)) in einem Molverhältnis von beispielsweise 2,0 bis 2,2, bezogen auf eingesetzte Verbindung der Formel (N) mit $R^{11}$ = Wasserstoff und b) bei der Alkylierung von vier N-Atomen (Herstellung von Verbindungen der Formeln (V) und VI)) in einem Molverhältnis von beispielsweise 4,0 bis 4,4, bezogen auf eingesetzte Verbindung der Formel (V) bzw. (VI) mit $R^{21}$ bzw. $R^{31}$ = Wasserstoff. Wenn das Alkylierungsmittel der Formel (VII) relativ wenig reaktiv ist, ist es im allgemeinen vorteilhaft, größere Mengen davon einzusetzen, im Falle a) z.B. 2,5 bis 3,5 Mol und im Falle b) z.B. 4,5 bis 8 Mol.

Die Indolderivate der Formel (I), in denen $R^4$ für einen Rest der Formel (III) steht, eignen sich auch als Farbbildner für druckkopierfähige, thermoreaktive und elektrochrome Aufzeichnungsmaterialien. Sie können auch in Mischung mit anderen bekannten Farbbildnern eingesetzt werden. Das Prinzip solcher Aufzeichnungsmaterialien ist bekannt aus den US-PS'en 2 800 457, 2 948 753, 3 096 189 und 3 193 404 und den

DE-OS'en 2 555 080, 2 700 937, 2 237 545, 3 203 059 und 3 337 296 und der EP-OS 0 108 382.

## Beispiele

### Beispiel 1

90 g eines Styrol/Butylmethacrylat-Copolymers wurden mit 7 g Ruß und 3 g eines Indolderivats der Formel (I) mit $R^1$ = n-$C_{16}H_{33}$, $R^2$ = $C_6H_5$, $R^3$ = $R^4$ = Wasserstoff und A = unsubstituierter Benzolring bei 150 °C verknetet. Nach Zerkleinerung, grober Vormahlung und anschließender Vermahlung in einer Strahlmühle wurde durch Sichtung ein Toner mit einer Partikelgröße $d_{50}$ von 13 µm erhalten. Die triboelektrische Aufladung gegenüber einem Roheisen-Carrier betrug 12,3 µC/g. Sie wurde von der Luftfeuchtigkeit praktisch nicht beeinflußt. Im Langzeit-Gerätetest wurde praktisch keine Abnahme der Aufladung festgestellt.

### Beispiel 2

90 g Styrol/Butylmethacrylat-Copolymer wurden mit 7 g Ruß und 3 g Polypropylen und 1,8g eines Indolderivats der Formel (I) mit $R^1$ = $CH_3$, $R^2$ = $C_6H_5$, $R^3$ = H, A = unsubstituierter Benzolring und $R^4$ = (II) mit $R^1$ = $CH_3$, $R^2$ = $C_6H_5$, $R^3$ = H, n = Null und B = unsubstituierter Benzolring bei 150 °C verknetet. Nach Zerkleinerung, grober Vormahlung und anschließender Vermahlung in einer Strahlmühle wurde durch Sichtung ein Toner mit einer Partikelgröße $d_{50}$ von 13 µm erhalten. Die triboelektrische Aufladung gegenüber einem Ferrit-Carrier betrug 15,7 µC/g. Sie wurde von der Luftfeuchtigkeit praktisch nicht beeinflußt. Im Langzeit-Gerätetest wurde keine Abnahme der Aufladung festgestellt.

### Beispiele 3 - 27

Analog den Beispielen 1 bzw. 2 wurden Toner mit anderen Indolderivaten hergestellt. Diese wiesen sehr ähnliche triboelektrische Eigenschaften auf.

Sofern $R^4$ für einen Rest der Formeln (II) oder (III) stand, war dieser Rest stets so substituiert, daß ein symmetrisches Gesamtmolekül der Formel (I) resultierte.

Tabelle 1

| Beispiel Nr. | eingesetztes Indolderivat der Formel (I) mit $R^3$ = H, B = einen Benzolring und | | | | | | Tonerherstellung nach Beispiel | Aufladung |
|---|---|---|---|---|---|---|---|---|
| | $R^1$ | $R^2$ | $R^4$ | $R^5$ | A | n bzw. m | | µC/g |
| 3 | $CH_3$ | $C_6H_5$ | $CH_3$ | - | unsubst.Benzolring | - | 1 | 8,9 |
| 4 | $CH_3$ | $C_6H_5$ | $C_2H_5$ | - | " | - | 1 | 14,3 |
| 5 | $CH_3$ | $C_6H_5$ | (II) | H | " | 1 | 1 | 12,6 |
| 6 | $CH_3$ | $C_6H_5$ | (II) | $NO_2$ | " | 0 | 1 | 12,1 |
| 7 | $CH_3$ | $C_6H_5$ | (II) | $N(CH_3)_2$ | " | 1 | 1 | 10,9 |
| 8 | $C_{12}H_{25}$ | p-Cl-$C_6H_4$ | H | - | 5-$CH_3$ | - | 2 | 12,1 |
| 9 | $CH_2C_6H_5$ | $C_6H_5$ | $C_4H_9$ | - | unsubst.Benzolring | - | 1 | 10,8 |
| 10 | $CH_2CH_2OCH_3$ | $C_6H_5$ | $C_8H_{17}$ | - | " | - | 1 | 8,5 |
| 11 | $C_4H_9$ | $C_6H_5$ | $C_6H_5$ | - | 5-Cl | - | 2 | 9,2 |
| 12 | Cyclohexyl | p-$CH_3O$-$C_6H_4$ | $C_{16}H_{33}$ | - | unsubst.Benzolring | - | 1 | 13,7 |
| 13 | $C_2H_5$ | $C_6H_5$ | (II) | H | 5-$CH_3$ | 0 | 1 | 11,3 |
| 14 | $C_4H_9$ | $CH_3$ | (II) | H | unsubst.Benzolring | 1 | 2 | 14,8 |
| 15 | $C_{22}H_{45}$ | $C_6H_5$ | (II) | CN | " | 0 | 1 | 13,0 |
| 16 | $CH(CH_3)_2$ | $C_6H_5$ | $C_2H_5$ | - | " | - | 1 | 12,5 |

Tabelle 1 (Fortsetzung)

| Beispiel Nr. | eingesetztes Indolderivat der Formel (I) mit $R^3 = H$, B = einen Benzolring und | | | | | | Tonerherstellung nach Beispiel | Aufladung |
|---|---|---|---|---|---|---|---|---|
| | $R^1$ | $R^2$ | $R^4$ | $R^5$ | A | n bzw. m | | µC/g |
| 17 | $CH_3$ | o-$CH_3OC_6H_5$ | $CH(C_2H_5)(C_4H_9)$ | - | unsubst.Benzolring | - | 2 | 13,9 |
| 18*) | $CH_3$ | $C_6H_5$ | $C_4H_9$ | - | " | - | 1 | 11,4 |
| 19*) | $CH_3$ | $C_6H_5$ | (II) | $N(CH_3)_2$ | " | 1 | 2 | 12,3 |
| 20 | H | $C_6H_5$ | $C_2H_5$ | - | " | - | 1 | 8,9 |
| 21*) | $C_4H_9$ | $CH_3$ | (II) | H | " | 0 | 1 | 8,4 |
| 22 | $CH_3$ | $C_6H_5$ | (III) | - | " | 1 | 1 | 10,1 |
| 23 | $CH_3$ | p-$CH_3OC_6H_4$ | (III) | - | " | 2 | 1 | 11,9 |
| 24 | $C_6H_5$-$CH_2$ | $C_6H_5$ | (III) | - | " | 4 | 2 | 9,8 |
| 25 | $CH_3$ | $C_6H_5$ | (III) | - | " | 8 | 1 | 11,8 |
| 26 | $C_4H_9$ | $C_6H_5$ | (III) | - | 5-$CH_3$ | 2 | 1 | 10,5 |
| 27 | NC-$(CH_2)_2$- | $C_6H_5$ | (III) | - | unsubst.Benzolring | 14 | 1 | 9,7 |

*) in diesen Beispielen war $R^3 = CH_3$

**Beispiel 28**

123 g eines Indolderivates der Formel (IV) mit $R^{11} = R^{13}$ = Wasserstoff und $R^{12}$ = Phenyl und 5 g Tetrabutylammoniumbromid wurden in 800 ml Toluol gelöst. Bei 90 °C wurden innerhalb einer Stunde 300 ml 50 Gew.-% wäßrige Natronlauge zugetropft, danach während 30 Minuten 186 g 1-Bromhexadecan. Es wurde 4 Stunden bei 90 °C nachgerührt. Danach wurde die wäßrige Phase abgetrennt und die organische

Phase viermal mit je 400 ml Wasser gewaschen. Dann wurde aus der organischen Phase das Toluol in Vakuum abgezogen und der ölige Rückstand mit 1,2 l Ethanol versetzt. Ein Teil des Ethanols wurde zusammen mit restlichem Toluol im Vakuum abgezogen. Nach dem Abkühlen wurde abgesaugt. Nach dem Trocknen im Vakuum wurden 224,5 g eines farblosen Pulvers vom Schmelzpunkt 51-53°C und der Formel (III) mit $R^{11}$ = Hexadecyl, $R^{12}$ = Phenyl und $R^{13}$ = Wasserstoff erhalten.

**Beispiele 29 - 36**

Auf analoge Weise wie Beispiel 28 wurden folgende Indolderivate der Formel (IV) hergestellt (siehe Tabelle 2)

**Tabelle 2**

**Beispiel 29**

$R^{11}$ = $C_{12}H_{25}$, $R^{12}$ = p-$C_6H_4Cl$, $R^{13}$ = H, A in 5-Stellung Methyl. Schmelzpunkt: 101°C

**Beispiel 30**

$R^{11}$ = $CH_2C_6H_5$, $R^{12}$ = $C_6H_5$, $R^{13}$ = $C_4H_9$, A unsubstituiert. Schmelzpunkt: 141°C

**Beispiel 31**

$R^{11}$ = $C_4H_9$, $R^{12}$ = $C_6H_5$, $R^{13}$ = $C_6H_5$, A in 5-Stellung Cl.

**Beispiel 32**

$R^{11}$ = $C_4H_9$, $R^{12}$ = $CH_3$, $R^{13}$ = H, A unsubstituiert.

**Beispiel 33**

$R^{11}$ = $CH_3$, $R^{12}$ = $C_6H_5$, $R^{13}$ = $CH_3$, A unsubstituiert.
Schmelzpunkt: 184 bis 185°C

**Beispiel 34**

$R^{11}$ = $C_{22}H_{45}$, $R^{12}$ = $C_6H_5$, $R^{13}$ = H, A unsubstituiert.

**Beispiel 35**

$R^{11}$ = $C_2H_5$, $R^{12}$ = $C_6H_5$, $R^{13}$ = H, A unsubstituiert.
Schmelzpunkt: 160°C

**Beispiel 36**

$R^{11}$ = $CH_3$, $R^{12}$ = $C_6H_5$, $R^{13}$ = $C_2H_5$, A unsubstituiert.
Schmelzpunkt: 168°C

**Beispiel 37**

42,8 g 1-Methyl-2-phenylindol und 11,5 g Dodecandisäure wurden in 100 ml Toluol bei 70-80°C mit 23 ml Phosphoroxichlorid während 1/2 h versetzt. Nach 9 h bei dieser Temperatur wurde die Lösung auf 300 ml 20-proz. Natronlauge ausgetragen. Die Wasserphase wurde bei 60-70°C abgetrennt. Beim Abkühlen fielen aus der Toluolphase 36,7 g (75 % d.Th.) farbl. Pulver vom Schmelzpunkt 190°C und der Formel (VI) mit $R^{31}$ = Methyl, $R^{32}$ = Phenyl, m = 8 und Ring C unsubstituiert aus.

**Beispiel 38 bis 40**

Analog Beispiel 37 wurden die Beispiele 38 bis 40 sowie die in Tabelle 1 unter Beispiel 22, 23, 24, 26 und 27 der genannten Verbindungen hergestellt.

**Beispiel 38**

$R^{31}$ = Methyl, $R^{32}$ = Phenyl, m = 2, Ring C unsubstituiert.
Schmelzpunkt: 273 ° C

**Beispiel 39**

$R^{31}$ = Wasserstoff, $R^{32}$ = Phenyl, m = 4, Ring C unsubstituiert.
Schmelzpunkt: >250 ° C

**Beispiel 40**

$R^{31}$ = Benzyl, $R^{32}$ = Phenyl, m = 4, Ring C unsubstituiert.
Schmelzpunkt: 174 ° C

**Beispiel 41**

3 g des Indolylderivats hergestellt gemäß Beispiel 37 wurden in einem Gemisch aus 40 g Dodecylbenzol und 60 g Chlorparaffin (45 Gew.-% Chlorgehalt) gelöst, 223 g einer solchen Lösung wurden mit 39,5 g Oxadiazintrion auf der Basis von Hexamethylendiisocyanat (NCO-Gehalt 20,5 Gew.-%) vermischt. Anschließend erfolgte die Vermischung mit 320 g 0,5 gew.-%iger wäßriger Polyvinylalkohollösung und die Emulgierung in einem Rotor/Stator-Emulgiergerät. Die Mikrokapselherstellung erfolgte durch Zusatz von 76 g 9,0 gew.-%iger wäßriger Diethylentriaminlösung. Eine Nachbehandlung erfolgte durch Erwärmen der erhaltenen Mikrokapseldispersion auf 60 ° C und 3-stündigem Rühren bei dieser Temperatur. Es wurde dabei eine 40 Gew.-% Mikrokapseln enthaltende wäßrige Dispersion mit Kapseln einer Größe von durchschnittlich 7,3 µm erhalten. Die Kapseln enthielten das Indolylbenzoxazin gelöst in Dodecylbenzol/Chlorparaffin.

250 ml dieser Mikrokapseldispersion wurden vorgelegt und unter intensivem Rühren 40 g Cellulosefeinschliff langsam eingestreut. Nach 50-minütigem intensivem Rühren erfolgte die Zugabe von 40 ml eines 50 gew.-%igen Styrol-Butadien-Rubber-Latex (Baystal® D 1600 der Fa. Bayer AG). Die resultierende, 48,5 gew.-%ige Streichfarbe wurde mit Wasser auf 30 Gew.-% Feststoffgehalt verdünnt und mit einer Luftbürste auf die Rückseite eines handelsüblichen Basispapiers gestrichen. Der Auftrag betrug (nach dem Trocknen bestimmt) 5 g/m$^2$.

Das so bestrichene Papier wurde mit der beschichteten Seite auf die mit säureaktiviertem Bentonit als Entwicklersubstanz beschichtete Seite eines handelsüblichen kohlefreien Durchschreibepapiers gelegt. Beim Schreiben auf das mit Kaspeln beschichtete Papier ergab sich auf dem Durchschreibepapier eine intensive blaugraue Durchschrift, die sehr lichtecht war.

Wurde das mit Mikrokapseln beschichtete Papier mit Tageslicht belichtet und schließend auf das zweite Blatt geschrieben, so wurde eine ebenso intensive Durchschrift erhalten.

Praktisch gleiche Ergebnisse wurden erhalten, wenn anstelle von säureaktiviertem Bentonit ein p-tert,-Butylphenyol-Formaldehyd-Kondensationsprodukt (UCAR®-CKWA 9870 der Fa. UCC) oder p-Alkyl-Zink-Salicylat als Entwickler verwendet wurde.

**Beispiel 42**

In einer Kugelmühle wurden 32 g Bisphenol A, 3,8 g Distearylamid des Ethylendiamins, 89 g Kaolin, 20 g eines zu 88 % hydrolysierten Polyvinylalkohols und 55 ml Wasser miteinander vermahlen, bis die mittlere Teilchengröße des Festsubstanzen 5 µm betrug. In einer zweiten Kugelmühle wurden 6 g des gemäß Beispiel 40 hergestellten Indolylderivats, 3 g eines zu 88 Gew.-% hydrolysierten Polyvinylalkohols und 60 ml Wasser miteinander vermahlen, bis die mittlere Teilchengröße des Feststoffs 3 µm betrug. Die beiden Dispersionen wurden zusammengegeben und auf Papier gestrichen, so daß nach dem Trocknen eine Beschichtung von 5,5, g/m$^2$ vorlag. Durch in Kontakt bringen dieses Papiers mit einem handelsüblichen Thermoschreibkopf wurde an den erwärmten Stellen eine intensive blaustichig-schwarze Färbung erhalten, die eine gute Licht- und Sublimierechtheit aufwies.

Analog Beispiel 41 und 42 lassen sich alle Indolderivate der Beispiele 37 bis 40 und 22 bis 27 einsetzen. Man erhält graue bis schwarze Färbungen, die teilweise blau-, grün- oder rotstichig sind.

## Patentansprüche

1. Toner für die Elektrofotografie, dadurch gekennzeichnet, daß sie als Ladungskontrollsubstanzen Indolderivate der Formel (I) enthalten

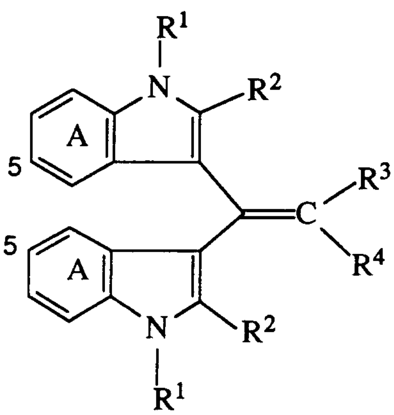
(I),

in der

$R^1$, $R^2$ und $R^3$ unabhängig voneinander für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl oder gegebenenfalls substituiertes $C_6$-$C_{10}$-Aryl stehen,

die Ringe A gegebenenfalls mit einem Benzolring anelliert und/oder gegebenenfalls mit bis zu 2 Resten aus der Gruppe $C_1$-$C_8$-Alkyl, $C_1$-$C_8$-Alkoxy, Halogen, Cyano, Nitro oder $C_6$-$C_{10}$-Aryl substituiert sind und

$R^4$ für Wasserstoff, gegebenenfalls verzweigtes und/oder substituiertes $C_1$-$C_{22}$-Alkyl, $C_5$-$C_7$-Cycloalkyl, $C_7$-$C_{12}$-Aralkyl, gegebenenfalls substituiertes $C_6$-$C_{10}$-Aryl oder einen Rest der Formeln (II) oder (III) steht

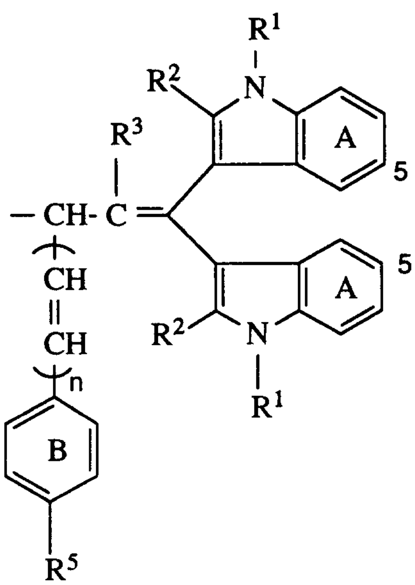
(II)

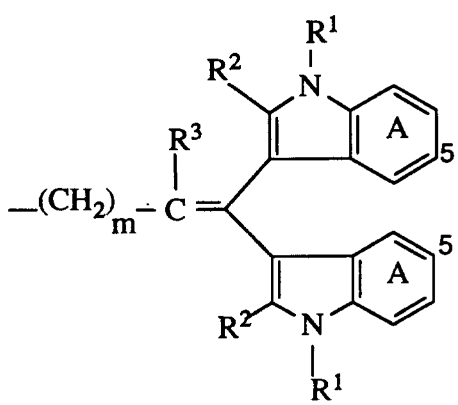
(III),

in denen

$R^1$, $R^2$, $R^3$ und A die unmittelbar zuvor angegebene Bedeutung haben,

$R^5$ für Wasserstoff, gegebenenfalls verzweigtes $C_1$-$C_8$-Alkyl, $C_5$-$C_7$-Cycloalkyl, gegebenenfalls verzweigtes $C_1$-$C_8$-Alkoxy, Halogen, Nitro, Cyano, $C_1$-$C_8$-Alkoxycarbonyl, $C_6$-$C_{10}$-Aryl oder Di-$C_1$-$C_8$-Alkylamino und
m für eine ganze Zahl von 1 bis 14 und
n für Null oder 1 stehen und
der Ring B unabhängig von den Ringen A außer mit $R^5$ gegebenenfalls wie die Ringe A anelliert und/oder substituiert ist.

**2.** Toner nach Anspruch 1, dadurch gekennzeichnet, daß in Formel (I)
$R^1$ für Wasserstoff, gegebenenfalls verzweigtes und/oder durch bis 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl oder Phenyl steht,
$R^2$ für gegebenenfalls verzweigtes und/oder durch bis zu 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl oder gegebenenfalls durch bis zu 2 Substituenten aus der Grupe $C_1$-$C_4$-Alkyl,$C_1$-$C_4$-Alkoxy, Chlor, Nitro und Cyano substituiertes Phenyl steht,
$R^3$ für Wasserstoff, Methyl oder Ethyl steht,
die Ringe A gegebenenfalls mit einem Benzolring anelliert oder gegebenenfalls mit bis zu 2 Resten aus der Gruppe Methyl, Ethyl, Methoxy, Chlor, Nitro, Cyano oder Phenyl substituiert sind und
$R^4$ für Wasserstoff, gegebenenfalls verzweigtes und/oder durch bis zu 3 Substituenten aus der Gruppe Chlor, $C_1$-$C_4$-Alkoxy und Cyano substituiertes $C_1$-$C_{22}$-Alkyl, Cyclopentyl, Cyclohexyl, Benzyl, gegebenenfalls durch bis zu 2 Substituenten aus der Gruppe $C_1$-$C_4$-Alkyl, $C_1$-$C_4$-Alkoxy, Chlor, Brom, Nitro und Cyano substituiertes Phenyl oder einen Rest der Formeln (II) oder (III) steht, in denen
$R^1$, $R^2$, $R^3$ und A die unmittelbar zuvor angegebene Bedeutung haben,
$R^5$ für Wasserstoff, gegebenenfalls verzweigtes $C_1$-$C_4$-Alkyl, Cycloalkyl, gegebenenfalls verzweigtes $C_1$-$C_4$-Alkoxy, Chlor, Brom, Nitro, Cyano, $C_1$-$C_4$-Alkoxycarbonyl, Phenyl oder Di-$C_1$-$C_4$-alkylamino und
m für eine ganze Zahl von 1 bis 14 und
n für Null oder 1 stehen und
der Ring B außer mit $R^5$ gegebenenfalls mit einem Benzolring anelliert oder gegebenenfalls bis zu zwei Resten aus der Gruppe Methyl, Ethyl, Methoxy, Chlor, Nitro, Cyano oder Phenyl substituiert ist.

**3.** Toner nach Ansprüchen 1 und 2, dadurch gekennzeichnet, daß in Formel (I)
$R^1$ für gegebenenfalls verzweigtes, unsubstituiertes $C_1$-$C_{22}$-Alkyl oder Benzyl steht,
$R^2$ für gegebenenfalls verzweigtes, unsubstituiertes $C_1$-$C_8$-Alkyl oder gegebenenfalls durch bis zu 2 Substituenten aus der Gruppe $C_1$-$C_4$-Alkyl, $C_1$-$C_4$-Alkoxy oder Chlor substituiertes Phenyl steht,
$R^3$ für Wasserstoff steht,
die Ringe A nicht anelliert, nicht substituiert oder in 5-Stellung durch Methyl, Methoxy oder Chlor substituiert sind und
$R^4$ für Wasserstoff, gegebenenfalls verzweigtes unsubstituiertes $C_1$-$C_{22}$-Alkyl oder einen Rest der Formeln (II) oder (III) steht,
in denen
$R^1$, $R^2$, $R^3$ und A die unmittelbar zuvor angegebene Bedeutung haben,
$R^5$ für Wasserstoff, gegebenenfalls verzweigtes $C_1$-$C_4$-Alkyl, gegebenenfalls verzweigtes $C_1$-$C_4$-Alkoxy, Chlor, Nitro, Cyano, Methoxycarbonyl oder Di-$C_1$-$C_4$-alkylamino und
m für eine ganze Zahl von 2 bis 8 und
n für Null oder 1 stehen und
der Ring B nicht anelliert oder außer mit $R^5$ nicht substituiert ist.

**4.** Toner nach Ansprüchen 1 bis 3, dadurch gekennzeichnet, daß sie 0,5 bis 3 Gew.-% Indolderivate der Formel (I) enthalten.

**5.** Indolderivate der Formeln (IV), (V) und (VI)

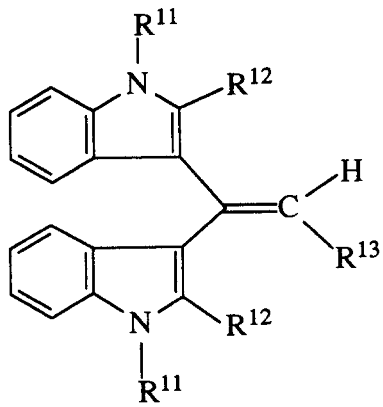

(IV)

in der

$R^{11}$ für gegebenenfalls verzweigtes und/oder gegebenenfalls durch bis zu 3 Substituenten aus der Gruppe Chlor, $C_1$-$C_4$-Alkoxy und Cyano substituiertes $C_5$-$C_{22}$-Alkyl steht und

entweder

$R^{12}$ für Phenyl, Tolyl, Anisyl oder Chlorphenyl und

$R^{13}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{16}$-Alkyl stehen

oder

$R^{12}$ für Tolyl, Anisyl oder Chlorphenyl und

$R^{13}$ für Wasserstoff oder gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{16}$-Alkyl stehen,

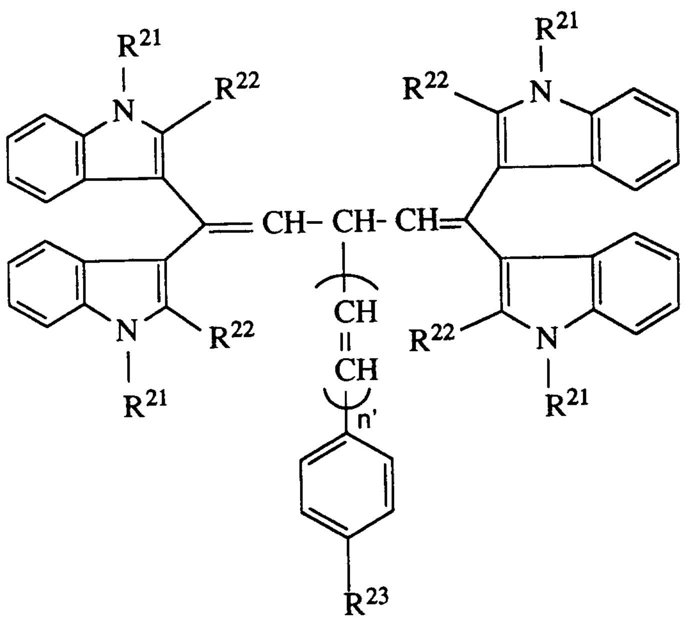

(V),

in der

$R^{21}$ für gegebenenfalls verzweigtes und/oder gegebenenfalls durch bis zu 3 Substituenten aus der Gruppe Chlor, $C_1$-$C_4$-Alkoxy und Cyano substituiertes $C_5$-$C_{22}$-Alkyl,

$R^{22}$ für Phenyl, Tolyl, Anisyl oder Chlorphenyl,

$R^{23}$ für Wasserstoff, Methyl, Methoxy, Chlor, Nitro oder Dimethylamino und

n' für Null oder 1 stehen und

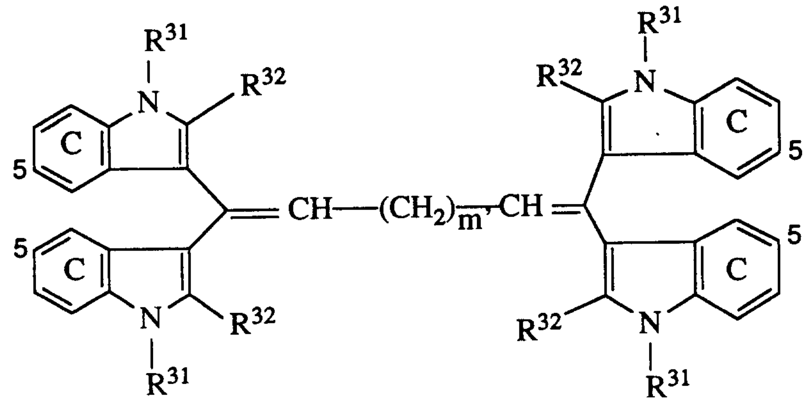

(VI),

in der

$R^{31}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_{22}$-Alkyl oder für Benzyl, Phenethyl, Cyclohexyl oder Cyclopentyl,

$R^{32}$ für gegebenenfalls verzweigtes und/oder gegebenenfalls durch bis zu 3 Substituenten aus der Gruppe Chlor, $C_1$-$C_4$-Alkoxy und Cyano substituiertes $C_1$-$C_8$-Alkyl oder für gegebenenfalls durch bis zu 3 Substituenten aus der Gruppe Chlor, $C_1$-$C_4$-Alkyl, $C_1$-$C_4$-Alkoxy und Cyano substituiertes Phenyl und

m' für eine ganze Zahl von 1 bis 14 stehen,

wobei die Ringe C gegebenenfalls mit einem Benzolring annelliert und/oder gegebenenfalls mit bis zu 2 Resten aus der Gruppe $C_1$-$C_8$-Aryl substituiert sind.

**6.** Indolderivate nach Anspruch 5, dadurch gekennzeichnet, daß

$R^{11}$ für gegebenenfalls verzweigtes, unsubstituiertes $C_5$-$C_{22}$-Alkyl steht,

im Falle $R^{12}$ = Tolyl, Anisyl oder Chlorphenyl $R^{13}$ für Wasserstoff oder unsubstituiertes $C_1$-$C_4$-Alkyl steht,

im Falle $R^{12}$ = Phenyl $R^{13}$ für unsubstituiertes $C_1$-$C_4$-Alkyl steht,

$R^{21}$ für gegebenenfalls verzweigtes, unsubstituiertes $C_5$-$C_{22}$-Alkyl steht,

$R^{31}$ für gegebenenfalls verzweigtes, unsubstituiertes $C_1$-$C_{22}$-Alkyl,

$R^{32}$ für gegebenenfalls verzweigtes unsubstituiertes $C_1$-$C_8$-Alkyl, Phenyl, Chorphenyl, Tolyl oder Phenethyl steht und

m' für eine ganze Zahl von 2 bis 8 steht, wobei

die Ringe C nicht annelliert, nicht substituiert oder in 5-Stellung durch $C_1$-$C_4$-Alkyl, $C_1$-$C_4$-Alkoxy oder Chlor substituiert sind.

**7.** Verfahren zur Herstellung von Indolderivaten des Anspruchs 5, dadurch gekennzeichnet, daß man Verbindungen der Formeln (IV), (V) oder (VI), bei denen $R^{11}$, $R^{21}$ bzw. $R^{31}$ für Wasserstoff steht, mit einem Alkylierungsmittel der Formel (VII) umsetzt

$R^{41}$ - X (VII),

in der

$R^{41}$ die bei der Formel (IV) für $R^{11}$, die bei Formel (V) für $R^{21}$ oder die bei Formel (VI) für $R^{31}$ angegebene Bedeutung hat und

X für Chlor, Brom, Iod, $OSO_2OR^{41}$, $OSO_2CH_3$, $OSO_2C_6H_5$, $OSO_2C_6H_4$-p-$CH_3$ oder $OSO_2C_6H_4$-p-Cl steht.

**8.** Verfahren nach Anspruch 7, dadurch gekennzeichnet, daß man die Umsetzung in einem Zwischenphasensystem durchführt, bei dem eine organische Phase und eine wäßrige Alkalilösung vorliegt.

**9.** Verwendung der Indolderivate nach Anspruch 1, in denen $R^4$ für einen Rest der Formel (III) steht, als Farbbildner für druckkopierfähige, thermoreaktive und elektrochrome Aufzeichnungsmaterialien.

Europäisches Patentamt

# EUROPÄISCHER RECHERCHENBERICHT

Nummer der Anmeldung

EP 93 10 4152

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 870 050 (HILTON DAVIS CO.) 26. September 1989 * Ansprüche 1,3,4,5,7-11,13,14 * | 1-6,9 | C07D209/08 C07D209/10 C07D209/12 C07D209/24 B41M5/136 B41M5/20 B41M5/30 |
| X | EP-A-0 325 708 (BAYER AG) 2. August 1989 * Ansprüche 4-6 * | 5,6 | |
| Y | EP-A-0 386 992 (APPLETON PAPERS INC.) 12. September 1990 * Ansprüche 1-4,9-13,15,19; Tabelle 3 * | 1-6,9 | |
| Y | EP-A-0 370 198 (BAYER) 30. Mai 1990 * gesamtes Dokument * | 1-6,9 | |

RECHERCHIERTE SACHGEBIETE (Int. Cl.5)

C07D
B41M

Der vorliegende Recherchenbericht wurde für alle Patentansprüche erstellt

| Recherchenort | Abschlußdatum der Recherche | Prüfer |
|---|---|---|
| MUENCHEN | 25 JUNI 1993 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)